# FASCICULE DE BREVET EUROPEEN

(11) **EP 2 091 509 B1**
(45) Date de publication et mention de la délivrance du brevet: **15.06.2016**
(21) Numéro de dépôt: 07870302.2
(22) Date de dépôt: 19.11.2007
(51) Int. Cl.: A61K 8/64, A61Q 7/00, A61K 38/47, A61Q 19/00, A61Q 19/08, C12Q 1/34, G01N 33/50, G01N 33/68

(54) **UTILISATION COSMÉTIQUE DE PROTÉINES DE TYPE CHITINASE**
KOSMETISCHE VERWENDUNG VON CHITINASEPROTEINEN
COSMETIC USE OF CHITINASE-TYPE PROTEINS

(30) Priorité: 20.11.2006 FR 0654986; 12.12.2006 US 874283 P
(43) Date de publication de la demande: 26.08.2009
(73) Titulaire: L'Oréal, 75008 Paris (FR)
(72) Inventeur: BERNARD, Dominique, 92170 VANVES (FR); DONOVAN, Mark, 95810 Berville (FR)
(74) Mandataire: Tanty, François
(86) Numéro de dépôt international: PCT/FR2007/001896
(87) Numéro de publication internationale: WO 2008/068428

(56) Documents cités:
- WO-A-00/34469
- WO-A-97/40068
- WO-A-2004/028479
- WO-A-2004/041170
- WO-A-2006/089549
- WO-A-2007/027748
- WO-A2-01/23430
- US-A- 6 060 590
- US-A1- 2002 086 008
- US-B1- 6 399 571

### SEQUENCE LISTING

<110> L'OREAL
<120> Utilisation cosmétique de protéines de type chitinase
<130> BR79971/CR/HG/klp
<140> 06 54986
   <141> 2006-11-20
<160> 6
<170> PatentIn version 3.1
<210> 1
   <211> 1152
   <212> DNA
   <213> Homo Sapiens
<400> 1
   atgggtgtga aggcgtctca aacaggcttt gtggtcctgg tgctgctcca gtgctgctct     60
   gcatacaaac tggtctgcta ctacaccagc tggtcccagt accgggaagg cgatgggagc    120
   tgcttcccag atgcccttga ccgcttcctc tgtacccaca tcatctacag ctttgccaat    180
   ataagcaacg atcacatcga cacctgggag tggaatgatg tgacgctcta cggcatgctc    240
   aacacactca agaacaggaa ccccaacctg aagactctct tgtctgtcgg aggatggaac    300
   tttgggtctc aaagattttc caagatagcc tccaacaccc agagtcgccg gactttcatc    360
   aagtcagtac cgccattcct gcgcacccat ggctttgatg ggctggacct tgcctggctc    420
   taccctggac ggagagacaa acagcatttt accaccctaa tcaaggaaat gaaggccgaa    480
   tttataaagg aagcccagcc agggaaaaag cagctcctgc tcagcgcagc actgtctgcg    540
   gggaaggtca ccattgacag cagctatgac attgccaaga tatcccaaca cctggatttc    600
   attagcatca tgacctacga ttttcatgga gcctggcgtg ggaccacagg ccatcacagt    660
   cccctgttcc gaggtcagga ggatgcaagt cctgacagat tcagcaacac tgactatgct    720
   gtggggtaca tgttgaggct gggggctcct gccagtaagc tggtgatggg catccccacc    780
   ttcgggagga gcttcactct ggcttcttct gagactggtg ttggagcccc aatctcagga    840
   ccgggaattc caggccggtt caccaaggag gcagggaccc ttgcctacta tgagatctgt    900
   gacttcctcc gcggagccac agtccataga accctcggcc agcaggtccc ctatgccacc    960
   aagggcaacc agtgggtagg atacgacgac caggaaagcg tcaaaagcaa ggtgcagtac   1020
   ctgaaggata ggcagctggc aggcgccatg gtatgggccc tggacctgga tgacttccag   1080
   ggctccttct gcggccagga tctgcgcttc cctctcacca atgccatcaa ggatgcactc   1140
   gctgcaacgt ag                                                       1152
<210> 2
   <211> 1089
   <212> DNA
   <213> Homo Sapiens
<400> 2
   tacaaactgg tctgctacta caccagctgg tcccagtacc gggaaggcga tgggagctgc 60
   ttcccagatg cccttgaccg cttcctctgt acccacatca tctacagctt tgccaatata 120
   agcaacgatc acatcgacac ctgggagtgg aatgatgtga cgctctacgg catgctcaac 180
   acactcaaga acaggaaccc caacctgaag actctcttgt ctgtcggagg atggaacttt 240
   gggtctcaaa gattttccaa gatagcctcc aacacccaga gtcgccggac tttcatcaag 300
   tcagtaccgc cattcctgcg cacccatggc tttgatgggc tggaccttgc ctggctctac 360
   cctggacgga gagacaaaca gcattttacc accctaatca aggaaatgaa ggccgaattt 420
   ataaaggaag cccagccagg gaaaaagcag ctcctgctca gcgcagcact gtctgcgggg 480
   aaggtcacca ttgacagcag ctatgacatt gccaagatat cccaacacct ggatttcatt 540
   agcatcatga cctacgattt tcatggagcc tggcgtggga ccacaggcca tcacagtccc 600
   ctgttccgag gtcaggagga tgcaagtcct gacagattca gcaacactga ctatgctgtg 660
   gggtacatgt tgaggctggg ggctcctgcc agtaagctgg tgatgggcat ccccaccttc 720
   gggaggagct tcactctggc ttcttctgag actggtgttg gagccccaat ctcaggaccg 780
   ggaattccag gccggttcac caaggaggca gggacccttg cctactatga gatctgtgac 840
   ttcctccgcg gagccacagt ccatagaacc ctcggccagc aggtccccta tgccaccaag 900
   ggcaaccagt gggtaggata cgacgaccag gaaagcgtca aaagcaaggt gcagtacctg 960
   aaggataggc agctggcagg cgccatggta tgggccctgg acctggatga cttccagggc 1020
   tccttctgcg gccaggatct gcgcttccct ctcaccaatg ccatcaagga tgcactcgct 1080
   gcaacgtag 1089
<210> 3
   <211> 63
   <212> DNA
   <213> Homo Sapiens
<400> 3
   atgggtgtga aggcgtctca aacaggcttt gtggtcctgg tgctgctcca gtgctgctct 60
   gca 63
<210> 4
   <211> 383
   <212> PRT
   <213> Homo Sapiens
<400> 4
   Met Gly Val Lys Ala Ser Gln Thr Gly Phe Val Val Leu Val Leu Leu
   1               5                   10                  15
   Gln Cys Cys Ser Ala Tyr Lys Leu Val Cys Tyr Tyr Thr Ser Trp Ser
               20                  25                  30
   Gln Tyr Arg Glu Gly Asp Gly Ser Cys Phe Pro Asp Ala Leu Asp Arg
           35                  40                  45
   Phe Leu Cys Thr His Ile Ile Tyr Ser Phe Ala Asn Ile Ser Asn Asp
       50                  55                  60
   His Ile Asp Thr Trp Glu Trp Asn Asp Val Thr Leu Tyr Gly Met Leu
   65                  70                  75                  80
   Asn Thr Leu Lys Asn Arg Asn Pro Asn Leu Lys Thr Leu Leu Ser Val
                   85                  90                  95
   Gly Gly Trp Asn Phe Gly Ser Gln Arg Phe Ser Lys Ile Ala Ser Asn
               100                 105                 110
   Thr Gln Ser Arg Arg Thr Phe Ile Lys Ser Val Pro Pro Phe Leu Arg
           115                 120                 125
   Thr His Gly Phe Asp Gly Leu Asp Leu Ala Trp Leu Tyr Pro Gly Arg
       130                 135                 140
   Arg Asp Lys Gln His Phe Thr Thr Leu Ile Lys Glu Met Lys Ala Glu
   145                 150                 155                 160
   Phe Ile Lys Glu Ala Gln Pro Gly Lys Lys Gln Leu Leu Leu Ser Ala
                   165                 170                 175
   Ala Leu Ser Ala Gly Lys Val Thr Ile Asp Ser Ser Tyr Asp Ile Ala
               180                 185                 190
   Lys Ile Ser Gln His Leu Asp Phe Ile Ser Ile Met Thr Tyr Asp Phe
           195                 200                 205
   His Gly Ala Trp Arg Gly Thr Thr Gly His His Ser Pro Leu Phe Arg
       210                 215                 220
   Gly Gln Glu Asp Ala Ser Pro Asp Arg Phe Ser Asn Thr Asp Tyr Ala
   225                 230                 235                 240
   Val Gly Tyr Met Leu Arg Leu Gly Ala Pro Ala Ser Lys Leu Val Met
                   245                 250                 255
   Gly Ile Pro Thr Phe Gly Arg Ser Phe Thr Leu Ala Ser Ser Glu Thr
               260                 265                 270
   Gly Val Gly Ala Pro Ile Ser Gly Pro Gly Ile Pro Gly Arg Phe Thr
           275                 280                 285
   Lys Glu Ala Gly Thr Leu Ala Tyr Tyr Glu Ile Cys Asp Phe Leu Arg
       290                 295                 300
   Gly Ala Thr Val His Arg Thr Leu Gly Gln Gln Val Pro Tyr Ala Thr
   305                 310                 315                 320
   Lys Gly Asn Gln Trp Val Gly Tyr Asp Asp Gln Glu Ser Val Lys Ser
                   325                 330                 335
   Lys Val Gln Tyr Leu Lys Asp Arg Gln Leu Ala Gly Ala Met Val Trp
               340                 345                 350
   Ala Leu Asp Leu Asp Asp Phe Gln Gly Ser Phe Cys Gly Gln Asp Leu
           355                 360                 365
   Arg Phe Pro Leu Thr Asn Ala Ile Lys Asp Ala Leu Ala Ala Thr
       370                 375                 380
<210> 5
   <211> 362
   <212> PRT
   <213> Homo Sapiens
<400> 5
   Tyr Lys Leu Val Cys Tyr Tyr Thr Ser Trp Ser Gln Tyr Arg Glu Gly
   1               5                   10                  15
   Asp Gly Ser Cys Phe Pro Asp Ala Leu Asp Arg Phe Leu Cys Thr His
               20                  25                  30
   Ile Ile Tyr Ser Phe Ala Asn Ile Ser Asn Asp His Ile Asp Thr Trp
           35                  40                  45
   Glu Trp Asn Asp Val Thr Leu Tyr Gly Met Leu Asn Thr Leu Lys Asn
       50                  55                  60
   Arg Asn Pro Asn Leu Lys Thr Leu Leu Ser Val Gly Gly Trp Asn Phe
   65                  70                  75                  80
   Gly Ser Gln Arg Phe Ser Lys Ile Ala Ser Asn Thr Gln Ser Arg Arg
                   85                  90                  95
   Thr Phe Ile Lys Ser Val Pro Pro Phe Leu Arg Thr His Gly Phe Asp
               100                 105                 110
   Gly Leu Asp Leu Ala Trp Leu Tyr Pro Gly Arg Arg Asp Lys Gln His
           115                 120                 125
   Phe Thr Thr Leu Ile Lys Glu Met Lys Ala Glu Phe Ile Lys Glu Ala
       130                 135                 140
   Gln Pro Gly Lys Lys Gln Leu Leu Leu Ser Ala Ala Leu Ser Ala Gly
   145                 150                 155                 160
   Lys Val Thr Ile Asp Ser Ser Tyr Asp Ile Ala Lys Ile Ser Gln His
                   165                 170                 175
   Leu Asp Phe Ile Ser Ile Met Thr Tyr Asp Phe His Gly Ala Trp Arg
               180                 185                 190
   Gly Thr Thr Gly His His Ser Pro Leu Phe Arg Gly Gln Glu Asp Ala
           195                 200                 205
   Ser Pro Asp Arg Phe Ser Asn Thr Asp Tyr Ala Val Gly Tyr Met Leu
       210                 215                 220
   Arg Leu Gly Ala Pro Ala Ser Lys Leu Val Met Gly Ile Pro Thr Phe
   225                 230                 235                 240
   Gly Arg Ser Phe Thr Leu Ala Ser Ser Glu Thr Gly Val Gly Ala Pro
                   245                 250                 255
   Ile Ser Gly Pro Gly Ile Pro Gly Arg Phe Thr Lys Glu Ala Gly Thr
               260                 265                 270
   Leu Ala Tyr Tyr Glu Ile Cys Asp Phe Leu Arg Gly Ala Thr Val His
           275                 280                 285
   Arg Thr Leu Gly Gln Gln Val Pro Tyr Ala Thr Lys Gly Asn Gln Trp
       290                 295                 300
   Val Gly Tyr Asp Asp Gln Glu Ser Val Lys Ser Lys Val Gln Tyr Leu
   305                 310                 315                 320
   Lys Asp Arg Gln Leu Ala Gly Ala Met Val Trp Ala Leu Asp Leu Asp
                   325                 330                 335
   Asp Phe Gln Gly Ser Phe Cys Gly Gln Asp Leu Arg Phe Pro Leu Thr
               340                 345                 350
   Asn Ala Ile Lys Asp Ala Leu Ala Ala Thr
           355                 360
<210> 6
   <211> 21
   <212> PRT
   <213> Homo Sapiens
<400> 6
   Met Gly Val Lys Ala Ser Gln Thr Gly Phe Val Val Leu Val Leu Leu
   1               5                   10                  15
   Gln Cys Cys Ser Ala
               20

## Description

La présente invention a pour objet la caractérisation d'un état d'un épiderme choisi parmi le vieillissement ou le photovieillissement.

Les épithéliums sont des tissus dont les cellules sont jointives et solidaires les unes des autres et reposent sur une membrane basale. Ils forment un revêtement soit externe, par exemple en surface de la peau, ou l'épiderme, soit interne, en surface d'une muqueuse. Ils peuvent également former des glandes.

L'épiderme est un épithélium, conventionnellement divisé en une couche basale de kératinocytes contenant, notamment, des cellules souches cutanées et constituant la couche germinative de l'épiderme, une couche dite épineuse constituée de plusieurs couches de cellules polyhédriques disposées sur la couche basale, une couche dite granuleuse comprenant une à trois couches dites de cellules aplaties contenant des inclusions cytoplasmiques distinctes, les grains de kératohyaline, et enfin, un ensemble de couches supérieures, appelé couche cornée (ou *stratum corneum*) constituée de kératinocytes au stade terminal de leur différenciation appelés cornéocytes.

Les épithéliums sont des structures dont l'homéostasie résulte de la mise en oeuvre d'un ensemble, finement régulé, de signaux intracellulaires et extracellulaires agissant à toutes les étapes de la prolifération, de la migration, de la différenciation cellulaire, ainsi que de la synthèse des différents composants de la matrice extracellulaire.

Ces signaux peuvent, notamment, résulter de l'action de facteurs produits par des kératinocytes.

Le maintien des bonnes fonctions physiologiques d'un épithélium implique notamment la différenciation épithéliale terminale et/ou la synthèse de protéoglycannes.

Dans le cas d'un épiderme par exemple, la différenciation épidermique suit un processus de maturation dans lequel des kératinocytes de la couche basale se différencient et migrent pour aboutir à la formation des cornéocytes, cellules mortes totalement kératinisées.

Cette différenciation est la résultante de phénomènes parfaitement coordonnés qui vont conduire au maintien d'une épaisseur constante et assurer ainsi l'homéostasie de l'épiderme.

De nombreux troubles ou pathologies cutanées peuvent résulter d'un dysfonctionnement de l'homéostasie de l'épithélium, et notamment de la différenciation épithéliale terminale des kératinocytes et/ou de la synthèse des protéoglycannes.

Dans le cas d'un épiderme, ces troubles peuvent se traduire, par exemple, par différentes atteintes de la peau âgée, résultant soit d'un vieillissement de l'épiderme dû au passage du temps (vieillissement chronologique) soit d'un vieillissement dû aux expositions à différents phénomènes extérieurs, comme une exposition au soleil (photovieillissement).

Ils peuvent également se traduire par des désordres de la fonction barrière de la peau contre les microorganismes, des hyperkératoses, une xérose (ou sécheresse cutanée), des ichtyoses, un psoriasis, des désordres de la desquamation, certaines lésions tumorales bénignes ou malignes, ou des affections des follicules pileux.

Toutefois, la méconnaissance des mécanismes physiologiques intimes et de l'ensemble des signaux intracellulaires et extracellulaires impliqués dans l'homéostasie des épithéliums, la différenciation terminale des kératinocytes et la synthèse des protéoglycannes rend difficile la préparation de compositions cosmétiques ou thérapeutiques qui soient susceptibles d'être efficacement mises en oeuvre dans le traitement des troubles épithéliaux, et notamment cutanées, précités.

Le polypeptide YKL-40 (YKL se référant à la séquence N-terminale du polypeptide mature SEQ ID NO 5), également connue sous le nom Human Cartilage glycoprotein 39 (HC gp-39) est un polypeptide de 383 acides aminés (SEQ ID NO 4 ; P36222, www.ncbi.nlm.nih.gov), isolé initialement à partir de culture de chondrocytes articulaires humains, et dont la maturation, par élimination de la séquence signal formée des 21 premiers acides aminés (SEQ ID NO 6), conduit à la libération extracellulaire du polypeptide identifié par la séquence SEQ ID NO 5 (Hakala et al., J. Biol. Chem., 1993, 268:25803).

Outre sa présence au niveau des chondrocytes articulaires, cette protéine a également été détectée dans d'autres tissus ou organes tel que les synoviocytes, les neutrophiles, les macrophages, le liquide synovial de patients atteints de polyarthrite rhumatoïde, le foie ou la rétine.

Par ailleurs le document WO2004/028479 enseigne la détection de YKL-40 dans des tissus atteints de psoriasis. WO2004/041170 enseigne la détection de YKL-40 dans des tissus de mammifères pour le diagnostic de maladies auto-immunes. US2002/0086008 enseigne des kits de diagnostic d'une infection, comprenant YKL-40 ou un anticorps anti-YKL-40.

YKL-40 est une glycoprotéine présentant un poids moléculaire apparent variant de 38 à 40 kDa selon la technique de mesure mise en oeuvre.

Cette protéine (ou polypeptide) est caractérisée par la présence de nombreuses similarités de séquences avec des chitinases d'origine bactériennes et/ou fongiques. Toutefois, elle ne paraît posséder ni activité chitinolytique, ni activité glycosidase.

Certaines fonctions physiologiques de la protéine YKL-40 sont notamment discutées dans les documents suivants : MALINDA et al., Exp. Cell Res., 1999, 250:168 ; DE CEUNINCK et al., Biochem. Biophys. Res. Commun, 2001, 285:926, RECKLIES et al., Biochem. J., 2002, 365:119, REHLI et al., J. Biol. Chem., 2003, 278:44058; SHACKELTON et al., J. Biol. Chem., 1995, 270:13076.

Pour sa part, la demande WO 03/07545 propose des compositions d'application topique associant à des glycosaminoglycannes ou à l'acide hyaluronique, la protéine HC-gp39 afin d'améliorer l'effet bénéfique des premiers composés sur une peau affectée par le vieillissement ou des traumatismes, par exemple de nature chirurgicaux.

Quant au brevet US 6,060,590, il décrit l'utilisation de protéines de type chitinase pour favoriser la cicatrisation de tissu endommagé, et notamment de la peau.

De manière inattendue, les inventeurs ont constaté que l'expression de la protéine YKL-40 par les kératinocytes de différentes zones cutanées d'un individu peut caractériser l'état d'un épiderme choisi parmi le vieillissement et le photovieillissement de cet épiderme.

Selon un de ses aspects, la présente invention concerne l'utilisation *in vitro* ou *ex vivo* d'au moins un polypeptide de séquence d'acides aminés codée par une séquence d'acides nucléiques choisie parmi SEQ ID NO 1, SEQ ID NO 2, ou d'au moins une séquence d'acides nucléiques codant pour ledit polypeptide à titre d'outil de caractérisation d'un état d'un épiderme, ledit état à évaluer étant choisi parmi le vieillissement ou le photovieillissement.

Par signes cutanés du vieillissement on entend toutes modifications de l'aspect extérieur de la peau dues au vieillissement qu'il soit chronobiologique et/ou photo-induit, comme par exemple les rides et ridules, la peau flétrie, le manque d'élasticité et/ou de tonus de la peau, l'amincissement du derme et/ou la dégradation des fibres de collagène ce qui entraîne l'apparence de peau molle et ridée. On entend également toutes les modifications internes de la peau qui ne se traduisent pas systématiquement par un aspect extérieur modifié, comme par exemple toutes les dégradations internes de la peau, particulièrement des fibres d'élastine, ou fibres élastiques, consécutives à une exposition aux rayonnements ultra-violets.

Selon un autre de ses aspects, la présente invention concerne un procédé de caractérisation d'un état d'un épiderme comprenant au moins les étapes consistant à :
a) déterminer, dans un échantillon dudit épiderme, une teneur en polypeptide de séquence d'acides aminés codée par une séquence d'acides nucléiques choisie parmi SEQ ID NO 1, SEQ ID NO 2, ou une séquence d'acides nucléiques codant pour ledit polypeptide, et
b) comparer ladite teneur déterminée à l'étape a) à une valeur de référence, ledit état à évaluer étant choisi parmi le vieillissement ou le photovieillissement.

### DEFINITION POLYPEPTIDE

Un polypeptide convenant à l'invention a une séquence d'acides aminés représentée en tout ou partie par une séquence choisie parmi SEQ ID NO 4 ou SEQ ID NO 5.

Au sens de la présente invention, on entend désigner de manière générale, sauf indications contraires, par YKL-40, la forme pro-protéine (SEQ ID NO 4) et la forme mature du polypeptide (SEQ ID NO 5).

Par « séquence caractéristique du polypeptide », on entend viser notamment au regard de YKL-40, la séquence pro-protéine constituée par la séquence SEQ ID NO 4, la séquence de la protéine mature identifiée par SEQ ID NO 5.

Selon un mode de réalisation, les polypeptides identifiés par les séquences SEQ ID NO 4 et SEQ ID NO 5 sont préférés pour la mise en oeuvre de la présente invention.

Un polypeptide convenant également à la mise en oeuvre de la présente invention peut être un polypeptide ayant subi une ou plusieurs modification(s) post-traductionnelle(s).

Par « modification(s) post-traductionnelle(s) », on entend englober l'ensemble des modifications qu'un peptide ou une protéine est susceptible de subir à l'issue de sa synthèse dans une cellule, telle que par exemple une ou des phosphorylation(s), une ou des glycosylation(s), une ou des lipidation(s), telles qu'une farnésylation ou une palmitoylation, un réarrangement structural de type formation de ponts disulfures et/ou de type clivage à l'intérieur de la séquence peptidique.

Selon un mode de réalisation un polypeptide convenant à la mise en oeuvre de l'invention peut également être un polypeptide naturel ou synthétique, le cas échéant susceptible d'être obtenu après lyse enzymatique ou chimique de YKL-40 ou par synthèse chimique ou biologique ou par extraction à partir d'un tissu biologique, comme par exemple la peau, exprimant naturellement ou après transduction ce polypeptide, ainsi que les différentes formes post-traductionnelles de celui-ci, ou encore tout polypeptide naturel ou synthétique dont la séquence comprend totalement ou partiellement (en tout ou partie) une séquence d'acides aminés précitée, par exemple les variants et les analogues.

L'homme de l'art peut obtenir un polypeptide convenant à l'invention au moyen de procédés à base d'ADN recombinant, comme par exemple, ceux décrits dans le manuel «Molecular Cloning - A Laboratory Manual » (2ème édition), Sambrook et al., 1989, Vol. I-III, Coldspring Harbor Laboratory, Coldspring Harbor Press, NY, (Sambrook).

### DEFINITION SEQUENCES ACIDES NUCLEIQUES

Selon un mode de réalisation, la présente invention concerne la mise en oeuvre de séquences d'acides nucléiques codant pour un polypeptide convenant à l'invention dans les différentes utilisations et procédés convenant à l'invention.

Une séquence d'acides nucléiques convenant à l'invention peut être choisie parmi SEQ ID NO 1, SEQ ID NO 2, ou une séquence d'acides nucléiques codant pour un polypeptide convenant à l'invention.

Les séquences d'acides nucléiques peuvent être issues de toutes origines possibles à savoir soit animale, en particulier de mammifères et encore plus particulièrement humaine, soit végétale, soit de micro-organismes (virus, phages, bactéries entre autres) ou encore de champignons, sans préjuger du fait qu'elles soient présentes de manière naturelle ou non dans ledit organisme d'origine.

Ainsi, la présente invention se rapporte également à l'utilisation de séquences d'acides nucléiques, notamment d'acides désoxyribonucléiques, ou d'acides ribonucléiques codant pour un polypeptide convenant à l'invention.

Les séquences d'acides nucléiques selon l'invention peuvent notamment être utilisées pour préparer des séquences d'acides ribonucléiques correspondantes sens ou antisens.

Un procédé peut être effectué sur un échantillon cellulaire, obtenu soit d'une biopsie cutanée soit à partir de cellules en culture.

Avantageusement, à titre d'échantillon cellulaire, on peut mentionner les kératinocytes.

Avantageusement, un polypeptide mis en oeuvre dans un procédé selon la présente invention peut être la protéine YKL-40.

L'expression d'une séquence d'acide nucléique peut être déterminée, par exemple, au moyen de sondes oligonucléotidiques, par tout protocole connu de l'homme de l'art.

A titre d'exemple de méthodes de détection de séquence d'acides nucléiques, il peut être fait mention de la réaction de polymérisation en chaîne (PCR), de la réaction de polymérisation en chaîne transcriptase inverse (RT-PCR ou Q-PCR), du Northern blot, de la méthode ribonuclease protection assay, des méthodes avec des puces à ADN, des méthodes avec des puces transcriptomiques, des méthodes avec des puces à oligonucléotides, des méthodes d'hybridation in situ.

A titre d'exemple d'agents convenant à la détection d'une séquence d'acides nucléiques, et en particulier d'ARNm, il peut être fait mention de sonde d'acides nucléiques marquée pouvant s'hybrider à ladite séquence.

Une telle sonde d'acide nucléique peut être aisément obtenue par toute méthode connue de l'homme de l'art.

Ainsi, les séquences d'acides nucléiques conformes à l'invention peuvent être utilisées pour réaliser des amorces oligonucléotidiques sens et/ou antisens, qui s'hybrident dans des conditions de forte stringence à la séquence SEQ ID NO : 1, SEQ ID NO :2, ou un analogue de celle-ci.

L'expression d'une séquence d'acide nucléique conforme à l'invention peut être comparée à une valeur de référence obtenue, par exemple, en effectuant un procédé conforme à l'invention en l'absence de composé à tester.

La détermination d'une teneur en polypeptide conforme à l'invention peut être effectuée au moyen de toute méthode connue de l'homme de l'art.

A titre de méthodes de détection d'un polypeptide, il peut être fait mention du Western blot, du Slot blot, du Dot blot, des méthodes ELISA (Enzyme Linked Immuno-Sorbent Assay) de type singleplex ou multiplex, des méthodes de protéomiques ou de glycomiques, de coloration de polypeptides dans un gel de polyacrylamide par un colorant à base d'Argent, par le bleu de Coomassie ou par le SYPRO, de l'immunofluorescence, de l'absorption UV, de méthodes immunohistochimiques en microscopie classique, électronique ou confocal, de FRET (fluorescence resonance energy transfer/transfert d'énergie par résonance de fluorescence), des méthodes de TR-FRET (time resolved FRET/FRET en résolution de temps), des méthodes de FLIM (fluorescence lifetime imaging microscopy/imagerie par microscopie uu temps de fluorescence), des méthodes de FSPIM (fluorescence spectral imaging microscopy/imagerie par microscopie en spectre de fluorescence), des méthodes de FRAP (fluorescence recovery after photobleaching/recouvrement de fluorescence après photoblanchiement), des méthodes par gène reporteur, des méthodes d'AFM (atomic force microscopy/microscopie par force atomique), des méthodes de résonance plasmonique de surface, des méthodes de microcalorimétrie, des méthodes de cytométrie en flux, des méthodes de biosenseurs, des méthodes de radioimmuno-essais (RIA), des méthodes d'isoelectric focusing, et des tests enzymatiques, des méthodes mettant en oeuvre des puces à peptides, des puces à sucre, des puces d'anticorps, des méthodes de spectrométrie de masse, des méthodes de spectrométrie de type SELDI-TOF (Ciphergen).

Un procédé conforme à l'invention peut être effectué sur un échantillon d'épiderme, obtenu à partir d'une biopsie cutané ou d'un modèle cellulaire épithéliale, par exemple épidermique ou d'un prélèvement de surface notamment par adhésif (stripping) de *stratum corneum.*

Un état d'un épiderme peut être un état lié à un dysfonctionnement de la différenciation épithéliale terminale, en particulier épidermique, notamment des kératinocytes, et/ou d'un défaut de synthèse des protéoglycannes et/ou d'une altération des défenses épithéliales, et notamment cutanées, contre des microorganismes.

Un tel état peut être d'origine chronologique (i.e. lié au temps écoulé comme le vieillissement cutané) et/ou indicatif d'un trouble cutané, lié par exemple au photovieillissement.

Selon un aspect, la présente invention concerne l'utilisation *in vitro* ou *ex vivo* de séquence d'acides aminés codée par une séquence d'acides nucléiques choisie parmi SEQ ID NO 1, SEQ ID NO 2, ou d'au moins une séquence d'acides nucléiques codant pour ledit polypeptide à titre d'outil de caractérisation d'un épiderme, ledit état à évaluer étant choisi parmi le vieillissement ou le photovieillissement.

Selon un mode de réalisation, la présente invention concerne un procédé de caractérisation d'un état d'un épiderme comprenant au moins les étapes consistant à :
a) déterminer, dans un échantillon dudit épiderme, une teneur en polypeptide de séquence d'acides aminés codée par une séquence d'acides nucléiques choisie parmi SEQ ID NO 1, SEQ ID NO 2, ou d'une séquence d'acides nucléiques codant pour ledit peptide, et
b) comparer ladite teneur déterminée à l'étape a) à une valeur de référence, ledit état à évaluer étant choisi parmi le vieillissement ou le photovieillissement.

Un procédé selon l'invention peut être effectué sur un échantillon d'épiderme, prélevé à partir d'un modèle cellulaire épithélial, et notamment épidermique, ou d'une peau isolée reconstruite afin d'en qualifier l'état.

Le prélèvement d'un échantillon d'épiderme, peut se faire par toute méthode connue de l'homme de l'art.

Un procédé selon l'invention peut être effectué *in vitro* ou *ex vivo.*

Une valeur de référence peut être, par exemple, une teneur en polypeptide ou en séquence d'acides nucléiques déterminée sur un échantillon d'épiderme prélevé sur un épithélium, et notamment une peau normale, c'est-à-dire satisfaisante sur le plan physiologique à l'image par exemple d'une peau jeune.

La mesure d'une valeur de référence peut être effectuée parallèlement ou séquentiellement à la détermination de ladite teneur d'un polypeptide ou d'une séquence d'acides nucléiques.

Une comparaison d'une teneur déterminée avec une valeur de référence peut permettre d'évaluer une déviation par rapport à cette valeur.

L'analyse de l'intensité et/ou de la nature de cette déviation (négative ou positive) peut être informative de l'état de l'épiderme.

La caractérisation d'un état d'un épiderme peut être indicative d'un éventuel trouble cutané.

Selon un mode de réalisation, un procédé selon l'invention peut être mis en oeuvre dans un procédé de diagnostic *in vitro* ou *ex vivo* d'un trouble présumé d'un épiderme, chez un individu, choisi parmi le vieillissement et le photovieillissement.

Un polypeptide convenant à la mise en oeuvre d'un procédé selon l'invention peut être avantageusement la protéine YKL-40.

La détermination d'une teneur en polypeptide conforme à l'invention ou en acides nucléiques conformes à l'invention dans un échantillon d'épiderme peut être effectuée par tout protocole connu de l'homme de l'art.

A titre de méthodes de détection d'un polypeptide, il peut être fait mention de celles citées précédemment.

Ainsi, il peut être envisagé de détecter la présence d'un polypeptide conforme à l'invention au moyen d'un anticorps, le cas échéant sous une forme marquée.

Un anticorps susceptible d'être utilisé à titre d'outil d'évaluation d'un état d'un épiderme peut être obtenu par tout procédé connu de l'homme de l'art, tel que décrit dans « Antibodies: A Laboraory Manual », Cold Spring Harbor Laboratory Press, Cold Spring Harbor, N.Y. (1990).

Une séquence d'acide nucléique convenant à la mise en oeuvre d'un procédé selon l'invention peut être avantageusement une séquence d'acides nucléiques codant pour YKL-40, par exemple de type ARNm.

A titre d'exemple de méthodes de détection d'acides nucléiques conformes à l'invention, il peut être fait mention des méthodes citées précédemment.

Au sens de la présente invention, « un » doit se comprendre, sauf indication contraire, au sens de « au moins un ».

Les exemples figurant ci-après sont présentés à titre illustratif et non limitatif de l'invention.

### EXEMPLE I

### Analyse par ELISA d'échantillons par prélevés stripping vernis sur différentes zones cutanées d'un individu

Les analyses sont réalisées à partir de strippings vernis effectués sur différentes zones cutanées d'un individu : jambe, bras, front, paume et dos de la main.

Des carrés de 3X3 cm de nylon sont utilisés.

Les poudres acétoniques (PA) sont extraites dans du tampon PBS+0.1 % Triton X-100 à raison de 100 µl/mg de PA dans un potter.

Après centrifugation, les surnageants sont recueillis.

Les protéines sont dosées selon la méthode de Bradford avec le réactif Bradford de Bio-Rad. Le dosage du YKL-40 est réalisé avec le kit Metra YKL40 Elisa kit (Quidel) à partir de 20 µl de chaque extrait.

Les essais sont réalisés en duplicate.

Chaque résultat est rapporté aux taux de protéines.

La présence d'YKL40 est détectée et quantifiée aisément sur toutes les zones cutanées analysées.

### EXEMPLE II

### Immunohistochimie

La coupe de follicule pileux est de 5 µm.

L'anticorps primaire est un anticorps polyclonal de lapin obtenu chez Osteomedical [Réf 4815] et utilisé au 1:25.

L'anticorps secondaire est un anticorps anti-Ig de lapin développé chez le chèvre et couplé à la biotine, obtenu chez Dako et utilisé au 1:500.

Au final, une amplification par streptavidine et une révélation par 3-amino-9-éthyle carbazole (AEC) sont réalisées.

Une forte concentration en protéine YKL-40 est observée à la base du follicule.

### Listes de Séquences

**SEQ ID NO 1**
**SEQ ID NO 2**
**SEQ ID NO 3**
**SEQ ID NO 4**
**SEQ ID NO 5**
**SEQ ID NO 6**
   1 mgvkasqtgf wlvllqccs a

## Revendications

1. Utilisation *in vitro ou ex vivo* d'au moins un polypeptide de séquence d'acides aminés codée par une séquence d'acides nucléiques choisie parmi SEQ ID NO 1, SEQ ID NO 2, ou d'au moins une séquence d'acides nucléiques codant pour ledit polypeptide à titre d'outil de caractérisation d'un état d'un épiderme, ledit état à évaluer étant choisi parmi le vieillissement ou le photovieillissement.

2. Procédé *in vitro* ou *ex vivo* de caractérisation d'un état d'un épiderme comprenant au moins les étapes consistant à :
a) déterminer dans un échantillon dudit épiderme, une teneur en polypeptide de séquence d'acides aminés codée par une séquence d'acides nucléiques choisie parmi SEQ ID NO 1, SEQ ID NO 2, ou une séquence d'acides nucléiques codant pour ledit polypeptide, et
b) comparer ladite teneur déterminée à l'étape a) à une valeur de référence, ledit état à évaluer étant choisi parmi le vieillissement ou le photovieillissement.

## Patentansprüche

1. Verwendung, *in vitro* oder *ex vivo,* von mindestens einem Polypeptid von einer Aminosäuresequenz, die durch eine Nukleinsäuresequenz codiert wird, die ausgewählt ist aus SEQ ID NO 1, SEQ ID NO 2, oder mindestens einer Nukleinsäuresequenz, die das Polypeptid codiert, als Werkzeug zur Charakterisierung eines Zustands einer Haut, wobei der zu bewertende Zustand aus der Alterung oder Lichtalterung ausgewählt ist.

2. Verfahren, *in vitro* oder *ex vivo,* zur Charakterisierung eines Zustands einer Epidermis, umfassend mindestens die folgenden Schritte:
a) Bestimmen, in einer Probe der Epidermis, eines Gehalts an Polypeptid von einer Aminosäuresequenz, die durch eine Nukleinsäuresequenz codiert wird, die ausgewählt ist aus SEQ ID NO 1, SEQ ID NO 2, oder einer Nukleinsäuresequenz, die das Polypeptid codiert, und
b) Vergleichen des in Schritt a) bestimmten Gehalts mit einem Referenzwert, wobei der zu bewertende Zustand aus der Alterung oder Lichtalterung ausgewählt ist.

## Claims

1. An *in vitro* or *ex vivo* use of at least one polypeptide having an amino acid sequence encoded by a nucleic acid sequence selected from among SEQ ID NO 1, SEQ ID NO 2, or of at least one nucleic acid sequence encoding said polypeptide as tool for characterization of the condition of an epidermis, said condition to be evaluated being selected from among aging or photo-aging.

2. An *in vitro* or *ex vivo* method to characterize a condition of an epidermis, comprising at least the steps of:
a) determining in a sample of said epidermis a content of polypeptide having an amino acid sequence encoded by a nucleic acid sequence selected from among SEQ ID NO 1, SEQ ID NO 2, or a nucleic acid sequence encoding said polypeptide; and
b) comparing said content determined at step a) with a reference value, said condition to be evaluated being selected from among aging or photo-aging.
